# EUROPÄISCHE PATENTSCHRIFT

(11) **EP 1 427 689 B1**
(45) Veröffentlichungstag und Bekanntmachung des Hinweises auf die Patenterteilung: **30.12.2009**
(21) Anmeldenummer: 02774551.2
(22) Anmeldetag: 03.09.2002
(51) Int. Cl.: C07C 33/20, A01N 31/04

(54) **ANTIMIKROBIELL WIRKSAME 4-METHYL-4-ARYL-2-PENTANOLE, DEREN HERSTELLUNG UND VERWENDUNG**
4-METHYL-4-ARYL-2-PENTANOLS WITH AN ANTIMICROBIAL ACTION, PRODUCTION AND USE THEREOF
4-METHYL-4-ARYL-2-PENTANOLS A ACTION ANTIMICROBIENNE, PRODUCTION ET UTILISATION DESDITS COMPOSES

(30) Priorität: 05.09.2001 DE 10143434
(43) Veröffentlichungstag der Anmeldung: 16.06.2004
(73) Patentinhaber: Symrise GmbH & Co. KG, 37603 Holzminden (DE)
(72) Erfinder: SCHMAUS, Gerhard, 37671 Höxter-Bosseborn (DE); JOPPE, Holger, 37586 Dassel (DE)
(74) Vertreter: Eisenführ, Speiser & Partner
(86) Internationale Anmeldenummer: PCT/EP2002/009793
(87) Internationale Veröffentlichungsnummer: WO 2003/024907

(56) Entgegenhaltungen:
- EP-A- 0 032 659
- WO-A-96/19428
- DATABASE CA [Online] CHEMICAL ABSTRACTS SERVICE, COLUMBUS, OHIO, US; AN 92:185719, "Gas-chromatographic determination of the saturated vapor pressure of perfumes" XP002223516 & MASLOZHIROVAYA PROMYSHLENNOST, Nr. 2, 1980, Seiten 25-26,
- DATABASE CA [Online] CHEMICAL ABSTRACTS SERVICE, COLUMBUS, OHIO, US; AN 62:69211, "Glycolysis inhibition with aryl alcohols" XP002223517 & J. DENTAL. RES., Nr. 40, 1961, Seite 384

## Beschreibung

Die vorliegende Erfindung betrifft antimikrobiell wirksame 4-Methyl-4-aryl-2-pentanole der nachfolgenden Formel 1, Verfahren zur Herstellung dieser Verbindungen sowie deren Verwendung als antimikrobielle Wirkstoffe.

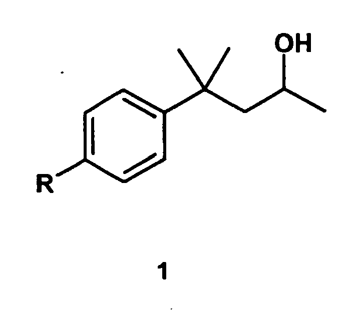

Als antimikrobiell wirksam haben sich überraschenderweise Verbindungen der Formel 1 erwiesen, für die gilt: R =
- Wasserstoff,
- Hydroxy,
- Alkoxygruppe mit bis zu 10 C-Atomen,
- geradkettiges oder verzweigtes, gesättigtes oder ungesättigtes Alkyl mit bis zu 10 C-Atomen,
- Alkylthioethergruppe mit bis zu 10 C-Atomen, wobei die Alkylthioethergruppe über eine Thioetherbrücke mit dem aromatischen Ring verbunden ist,
- Fluor, Chlor, Brom, lod oder
- durch ein oder mehrere Sauerstoff- und/oder Schwefelatome unterbrochenes Alkyl mit bis zu 10 C-Atomen.

Jede dieser Verbindungen sowie deren Gemische eignen sich als antimikrobielle Wirkstoffe bzw. Wirkstoffgemische
(a) zur kosmetischen Behandlung von Schuppen verursachenden Mikroorganismen,
(b) zur kosmetischen Behandlung von Körpergeruch verursachenden Mikroorganismen,
(c) zur kosmetischen Behandlung von Akne verursachenden Mikroorganismen
(d) zur kosmetischen Behandlung von Mykosen verursachenden Mikroorganismen.
(e) zur Behandlung von Mikroorganismen auf oder in unbelebter Materie, und/oder
(f) zur Konservierung verderblicher Artikel.

Die menschliche Haut wird von einer Vielzahl verschiedener Bakterien besiedelt. Die meisten dieser Bakterien sind nicht pathogen und für den physiologischen Zustand der Haut und für deren Geruch irrelevant. Andere hingegen können den gesunden Zustand der Haut maßgeblich beeinflussen. Einige, die menschliche Hautflora stark beeinflussende Mikroorganismen sind in Tabelle 1 dargestellt.

**Tabelle 1:**

| **Mikroorganismen:** | |
|---|---|
| | |
| *Staphylococcus epidermidis* | Achselgeruch; allg. Körpergeruch |
| *Staphylococcus aureus* | Atopische Ekzeme; Wundinfektion |
| *Corynebacterium xerosis* | Achselgeruch |
| *Brevibacterium epidermidis* | Achselgeruch; Fußgeruch |
| *Propionibacterium acnes* | Akne |
| *Escherichia coli* | Wundinfektionen |
| *Pseudomonas aeruginosa* | Wundinfektionen |
| *Malassezia furfur (syn. Pityrosporum ovale)* | Schuppenbildung |
| *Candida albicans* | Allg. Candidosen |
| *Trichophyton mentagrophytes* | Haut- und Nagelmykosen |
| *Trichophyton rubrum* | Haut- und Nagelmykosen |
| *Epidermophyton floccosum* | Haut- und Nagelmykosen |
| *Aspergillus niger* | Schimmelbefall |

Durch den bakteriellen Abbau im Schweiß enthaltener, körpereigener Stoffe wie z.B. ungesättigter Fettsäuren entstehen aus mehr oder minder schwach riechenden Vorstufen unangenehm riechende Zersetzungsprodukte, die das körperliche Wohlbefinden stark beeinflussen können. Zur Verhinderung der Entstehung der für Körpergeruch verantwortlichen Substanzen verwendet man in der Kosmetik Produkte, die entweder die Bildung von Körperschweiß unterbinden (sogenannte Antiperspirantien) oder Substanzen, die das Wachstum der für die Geruchsbildung verantwortlichen Bakterien der menschlichen Haut hemmen (Deodorantien). Bakterienarten wie *Staphylococcus epidermidis, Corynebacterium xerosis* und *Brevibacterium epidermidis* sind maßgeblich für die Bildung von Achsel- und Fußgeruch, bzw. Körpergeruch im allgemeinen verantwortlich. In der kosmetischen Industrie besteht daher ein beständiger Bedarf an neuen Mitteln zur Behandlung dieser und anderer Körpergeruch (einschließlich Achsel- und Fußgeruch) verursachenden Mikroorganismen.

Ein Akne verursachener Mikroorganismus ist *Propionibacterium acnes,* bei dem es sich um einen anaerob wachsenden Keim handelt. Die kosmetische Industrie sucht beständig nach Mitteln zur Behandlung dieses Keimes und anderer Akne verursachender Mikroorganismen.

Alle Bereiche der menschlichen Haut können von Mykosen (insbesondere Dermatomykosen und Nagelmykosen) befallen werden. Besonders häufig sind Hautareale betroffen, auf welchen sich durch das Tragen von Kleidung, Schuhwerk oder Schmuck Feuchtigkeit und Wärme stauen können. Als besonders unangenehm empfunden werden Pilzerkrankungen der Finger- und Fußnägelbereiche. Maßgeblich verantwortlich für die Bildung von Mykosen sind häufig verschiedene *Trichophyton-* und *Epidermophyton*-Arten. Die kosmetische Industrie sucht beständig nach neuen Mitteln zur Behandlung dieser und anderer Mykosen verursachenden Mikroorganismen.

Im Bereich der Haarpflege wird darüber hinaus intensiv nach Substanzen zur Behandlung des für die Schuppenbildung maßgeblich verantwortlichen Keimes *Malassezia furfur* und anderer Schuppen verursachenden Mikroorganismen gesucht.

Unter "Behandlung" wird dabei im Rahmen des vorliegenden Textes jede Form der Einflussnahme auf die betreffenden Mikroorganismen verstanden, bei der die Vermehrung dieser Mikroorganismen gehemmt und/oder die Mikroorganismen getötet werden.

In der kosmetischen und auch pharmazeutischen Industrie besteht zudem ein beständiger Bedarf an Mitteln zur Konservierung ansonsten verderblicher kosmetischer oder pharmazeutischer Artikel.

Bei der Suche nach entsprechenden antimikrobiell wirksamen und/oder konservierenden Mitteln ist dabei zu beachten, dass die in kosmetischen und/oder pharmazeutischen Produkten verwendeten Substanzen
- toxikologisch unbedenklich,
- gut hautverträglich,
- stabil (insbesondere in den üblichen kosmetischen und/oder pharmazeutischen Formulierungen),
- vorzugsweise geruchlos und
- preiswert herstellbar (d.h. unter Einsatz von Standardverfahren und/oder ausgehend von Standardprekursoren)
sein müssen.

Die Suche nach geeigneten (Wirk-)substanzen, die eine oder mehrere der genannten Eigenschaften in ausreichendem Maße besitzen, ist dem Fachmann dadurch erschwert, dass keine klare Abhängigkeit zwischen der chemischen Struktur einer Substanz einerseits und ihrer biologischen Aktivität gegenüber bestimmten Mikroorganismen (Keimen) sowie ihrer Stabilität andererseits besteht. Des Weiteren gibt es keinen vorhersehbaren Zusammenhang zwischen der antimikrobiellen Wirkung, der toxikologischen Unbedenklichkeit, der Hautverträglichkeit und/oder der Stabilität.

Es war daher die Aufgabe der vorliegenden Erfindung, einen antimikrobiellen Wirkstoff anzugeben, der zumindest gegen einen Mikroorganismus, vorzugsweise aber gegen mehrere der vorstehend diskutierten Mikroorganismen wirksam ist und dabei vorzugsweise auch eine oder mehrere der genannten Nebenbedingungen erfüllt.

Überraschenderweise hat sich nun gezeigt, dass die eingangs genannten 4-Methyl-4-aryl-2-pentanole mit den angegebenen Bedeutungen des Restes R hervorragende antimikrobielle Eigenschaften besitzen und gegen eine ganze Reihe von Mikroorganismen aktiv sind.

Unter den genannten Verbindungen der Formel 1 sind die Verbindungen 4-Methyl-4-phenyl-2-pentanol (CA-Nummem 2035-93-0, 2R/S-Gemisch; 329313-22-6, 2R-Isomer und 329313-20-4, 2S-Isomer) sowie 4-Methyl-4-p-cumenyl-2-pentanol (CA-Nummer: 93963-35-0); R = i-Propyl) bekannt. Über eine antimikrobielle Wirksamkeit der genannten Substanzen wurde bislang jedoch nicht berichtet. Aus J. Dental Res. 40,384 (1961) ist zudem bekannt, dass 4-Methyl-4-phenyl-2-pentanol eine bestimmte Glycolysehemmende Wirkung besitzt, weshalb sich diese Substanz als kariostatischer Wirkstoff eignet. Über eine antimikrobielle Wirksamkeit der genannten Substanzen wurde bislang jedoch nicht berichtet. Ferner sind aus Maslozhirovaya Promyshlennost (1980). (2), 25-6 Verbindungen der unten beschriebenen Formel 1 mit R = Wasserstoff bekannt.

Die Alkohole der Formel 1 besitzen eine starke antimikrobielle Wirkung gegenüber geruchsbildenden Mikroorganismen der menschlichen Haut und können somit hervorragend als Alternative oder als Ergänzung zu bekannten antimikrobiellen Wirkstoffen (wie z.B. Famesol) in Kosmetikprodukten und dergleichen als Deodorantien Einsatz finden. Die erfindungsgemäßen Verbindungen der Formel 1 sind auch gegenüber Propionibacterium acnes, Malassezia furfur und Mykosen verursachenden Keimen wie z.B. Trichophyton mentagrophytes gut wirksam, so dass sie auch zur Behandlung (Bekämpfung) von Akne, als Antischuppenmittel oder bei der Behandlung von Mykosen (insbesondere Dermatomykosen) eingesetzt werden können. Aufgrund ihres breiten Wirksamkeitsspektrums und insbesondere aufgrund ihrer Wirksamkeiten auch gegenüber gramnegativen Bakterien wie *Escherichia coli* und *Pseudomonas aeruginosa*, gegenüber Hefen wie *Candida albicans* und gegenüber Pilzen wie *Aspergillus niger* sind die erfindungsgemäßen Verbindungen der Formel 1 auch hervorrangende Mittel zur Konservierung beispielsweise kosmetischer und/oder pharmazeutischer Formulierungen.

Die Einsatzkonzentration eines erfindungsgemäßen Alkohols der Formel 1 in einem kosmetischen Endprodukt (z.B. einer Creme, einem Shampoo o.dgl.) liegt vorzugsweise im Bereich zwischen 0,0069 und 20 Gew.-%, bevorzugt im Bereich zwischen 0,05 und 5 Gew.-%, jeweils bezogen auf die Gesamtmasse des kosmetischen Produktes.

In einem bevorzugten Verfahren zur kosmetischen und/oder therapeutischen Behandlung von (a) Schuppen verursachenden Mikroorganismen, (b) Körpergeruch verursachenden Mikroorganismen (c) Akne verursachenden Mikroorganismen und/oder (d) Mykosen verursachenden Mikroorganismen wird eine antimikrobiell wirksame Menge einer oder mehrerer erfindungsgemäßen Verbindungen der Formel 1 topisch auf den menschlichen oder tierischen Körper appliziert, so dass das Wachstum des oder der ggf. anwesenden Mikroorganismen gehemmt und/oder diese abgetötet werden.

Die erfindungsgemäßen Verbindungen der Formel 1 sind aber selbstverständlich nicht nur zur Applikation auf den menschlichen oder tierischen Körper vorgesehen, sondern sind beispielsweise auch (e) zur Behandlung von Mikroorganismen auf oder in unbelebter Materie sowie (f) zur Konservierung verderblicher Artikel geeignet.

Eine erfindungsgemäße Substanz der Formel 1 kann auch als Bestandteil von Duftstoffkompositionen (Riechstoffkompositionen) eingesetzt werden und beispielsweise einem parfümierten Fertigprodukt eine antimikrobielle Wirkung verleihen. Eine besonders bevorzugte Duftstoffkomposition umfasst (a) eine sensorisch wirksame Menge eines Duftstoffes, (b) eine konservierend wirkende Menge einer oder mehrerer Verbindungen der Formel 1 (wobei R jede der oben angegebenen Bedeutungen besitzen kann) sowie (c) ggf. einen oder mehrere Trägerstoffe und/oder Zusatzstoffe. Da der Anteil an Parfüm in einem kosmetischen Fertigprodukt häufig im Bereich von ca. 1 Gew.-% liegt, wird ein Parfüm, welches eine erfindungsgemäße Verbindung der Formel 1 enthält, vorzugsweise zu etwa 5-50 Gew.-% aus einer oder mehreren Verbindungen der Formel 1 bestehen. Als besonders vorteilhaft hat es sich erwiesen, dass die Substanzen der Formel 1 nur einen schwachen Eigengeruch besitzen oder gar völlig geruchlos sind; denn diese Eigenschaft prädestiniert sie für den Einsatz als Konservierungsmittel in einer Duftstoffkomposition.

Die Erfindung betrifft auch antimikrobielle Zusammensetzungen, die neben (a) einer antimikrobiellen wirksamen Menge einer oder mehrerer erfindungsgemäßen Verbindungen der Formel 1 (wobei R jede der oben angegebenen Bedeutungen besitzen kann) auch (b) eine zu Komponente (a) kompatible Trägersubstanz umfasst.

Weitere Aspekte der vorliegenden Erfindung ergeben sich aus den beigefügten Patentansprüchen und den nachfolgenden Beispielen.

### 1. Synthese von 4-Methyl-4-arvl-2-pentanolen der allg. Formel 1

### Beispiel 1:

### Synthese von (R,S)-4-Methyl-4-phenyl-2-pentanol (Formelschema 1; Formel 2)

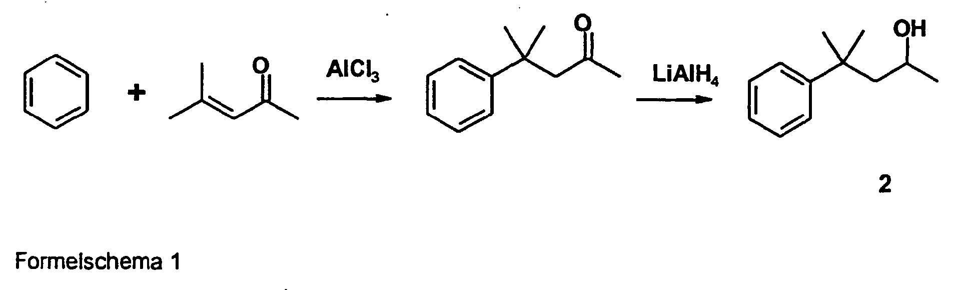

### a) Friedel-Crafts-Acylierung von Benzol mit Mesityloxid

In einem 21 Rührwerk werden 110,4g (0,83 mol) Aluminiumchlorid vorgelegt. Anschließend werden 360 ml (3,4 mol) Benzol zugesetzt. 68,6 g (0,7 mol) Mesityloxid werden unter Rühren und Kühlung bei ca. 10°C zugetropft . Der Reaktionsansatz wird 20h nachgerührt. Anschließend wird der Aluminiumkomplex bei einer Temperatur von max. 20 °C mit 100 ml Wasser hydrolysiert. Die wässrige Phase wird im Scheidetrichter abgetrennt. Anschließend wird nochmals mit 100 ml Wasser nachgewaschen. Die organische Phase wird abgetrennt und mit wässriger Natronlauge neutral gewaschen. Überschüssiges Benzol wird abdestilliert. Rohausbeute: 180g; Ausbeute an 4-Methyl-4-phenyl-2-pentanon 165g; Reinheit: 96%.

### b) Reduktion von 4-Methyl-4-phenyl-2-pentanon mit LiAlH₄

In einem 250 ml Kolben werden 50 ml absol. THF und 1,5g LiAlH₄ (39,5 mmol) vorgelegt. Bei ca. 0°C bis 5°C tropft man 18,5g 4-Methyl-4-phenyl-2-pentanone (105 mmol) gelöst in 10 ml absolutem THF zu. Der Reaktionsansatz wird 1 h nachgerührt. Anschließend wird das überschüssige LiAlH₄ durch Zusatz von Wasser hydrolysiert. Nach Verseifung mit 10% iger NaOH, neutralwaschen und abdestillieren des Lösungsmittels erhält man ca. 18g (R,S)-4-Methyl-4-phenyl-2-pentanol: Reinheit 97%.

Spektroskopische Daten von (R,S)-4-Methyl-4-phenyl-2-pentanol (Formel 2): ¹³C (CDCl₃; 75,5 MHz): δ (ppm) = 25.0 (q), 29.3 (q), 29.6 (q), 37.0 (s), 53.5 (t), 65.5 (d), 125.7 (d), 125.8 (2d), 128.2 (2d), 148.8 (s); MS: m/z (%) = 178 (-, M⁺), 160 (5), 145 (8), 120 (45), 119 (100), 118 (8), 117 (7), 105 (10), 91 (49), 79 (10), 78 (6), 77 (5).

### Beispiel 2:

### Synthese von (R,S)- 4-Methyl-4-p-tolyl-2-pentanol, (Formelschema 2; Formel 3)

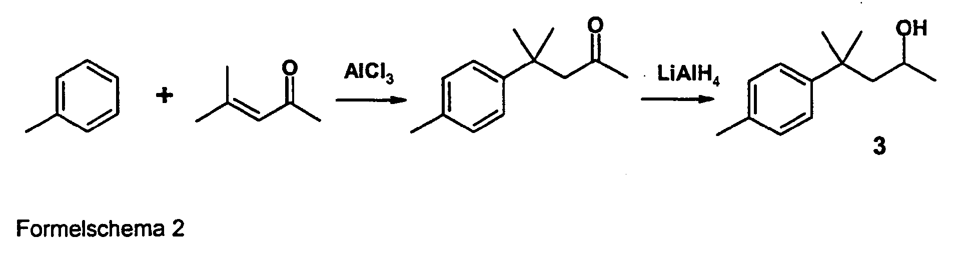

### a) Friedel-Crafts-Acylierung von Toluol mit Mesityloxid

In einem 2I Rührwerk werden 110,4g (0,83 mol) Aluminiumchlorid vorgelegt. Anschließend werden 312g (3,4 mol) Toluol zugesetzt. Unter Rühren und Kühlen werden 68,6 g (0,7 mol) Mesityloxid zudosiert. Der Reaktionsansatz wird 20h nachgerührt. Anschließend wird der Reaktionsansatz analog zu Beispiel 1 weiterverarbeitet. Rohausbeute: 115g; Ausbeute an 4-Methyl-4-p-tolyl-2-pentanon: 86g (Reinheit: 94%)

### b) Reduktion von 4-Methyl-4-p-tolyl-2-pentanon mit LiAlH₄

In einem 250 ml Kolben werden 50 ml absol. THF und 1,5g LiAlH₄ (39,5 mmol) vorgelegt. Bei ca. 0°C bis 5°C tropft man 20g 4-Methyl-4-p-tolyl-2-pentanone (105 mmol) gelöst in 10 ml absolutem THF zu. Der Reaktionsansatz wird 1 h nachgerührt. Anschließend wird das überschüssige LiAlH₄ durch Zusatz von Wasser hydrolysiert. Nach Verseifung mit 10% iger NaOH, neutralwaschen und abdestillieren des Lösungsmittels erhält man ca. 21g (R,S)-4-Methyl-4-p-tolyl-2-pentanol (Reinheit: 92%).

Spektroskopische Daten von (R,S)-4-Methyl-4-p-tolyl-2-pentanol (Formel 3): ¹³C (CDCl₃; 75,5 MHz): δ (ppm) = 20.8 (q), 24.9 (q), 29.0 (q), 30.0 (q), 36.7 (s), 53.5 (t), 65.5 (d), 125.7 (2d), 129.0 (2d), 135.1 (s), 145.7 (s); MS: m/z (%) = 192 (7, M⁺), 134 (29), 133 (100), 119 (10), 117 (6), 115 (6), 105 (27), 93 (7), 91 (9), 85 (1), 77 (3).

### Beispiel 3:

### Synthese von (R,S)-4-Methyl-4-p-cumenyl-2-pentanol (Formelschema 3; Formel 4)

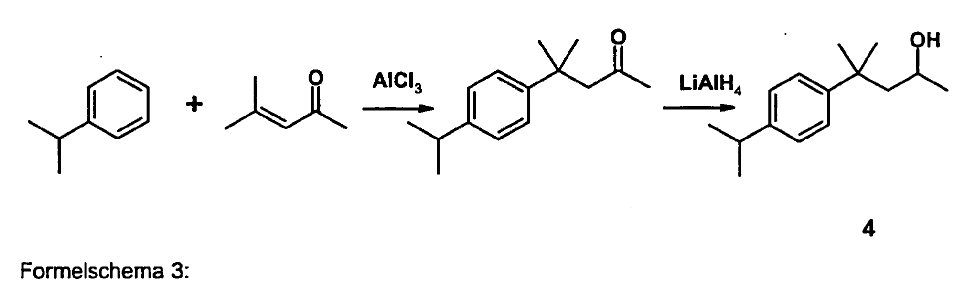

### a) Friedel-Crafts-Acylierung von Cumol mit Mesityloxid

In einem 21 Rührwerk Kolben werden 110,4g (0,83 mol) Aluminiumchlorid vorgelegt. Anschließend werden 408 g (3,4 mol) Cumol zugesetzt. Unter Rühren und Kühlen werden 68,6 g (0,7 mol) Mesityloxid zudosiert. Der Reaktionsansatz wird 20h nachgerührt. Anschließend wird der Reaktionsansatz analog zu Beispiel 1 weiterverarbeitet. Rohausbeute: 415g; Ausbeute an 4-Methyl-4-p-cumenyl-2-pentanon: 22g (Reinheit: 87%)

### b) Reduktion von 4-Methyl-4-p-cumenyl-2-pentanon mit LiAlH₄

In einem 250 ml Kolben werden 50 ml absol. THF und 0,5g LiAlH₄ (13,2 mmol) vorgelegt. Bei ca. 0°C bis 5°C tropft man 10g 4-Methyl-4-p-cumenyl-2-pentanon (45,8 mmol) gelöst in 10 ml absolutem THF zu. Der Reaktionsansatz wird 1 h nachgerührt. Anschließend wird das überschüssige LiAlH₄ durch Zusatz von Wasser hydrolysiert. Nach Verseifung mit 10% iger NaOH, neutralwaschen und abdestillieren des Lösungsmittels erhält man 9g (R,S)-4-Methyl-4-p-cumenyl-2-pentanol (Reinheit: 85%)

Spektroskopische Daten von (R,S)-4-Methyl-4-p-cumenyl-2-pentanol (Formel 4): ¹³C (CDCl₃; 75,5 MHz): δ (ppm) = 24.0 (2q), 24.8 (q), 29.0 (q), 30.0 (q), 33.4 (d), 36.7 (s), 53.6 (t), 65.4 (d), 125.7 (2d), 126.3 (2d), 145.9 (s), 146.0 (s); MS: m/z (%) = 220 (6, M⁺), 162 (22), 161 (100), 145 (7), 133 (4), 131 (4), 119 (20), 115 (3), 105 (8), 91 (11), 77 (2).

### Beispiel 4:

### Synthese von (R,S)-4-Methyl-4-(4-n-butylphenyl)-2-pentanol, (Formelschema 4), Formel 5

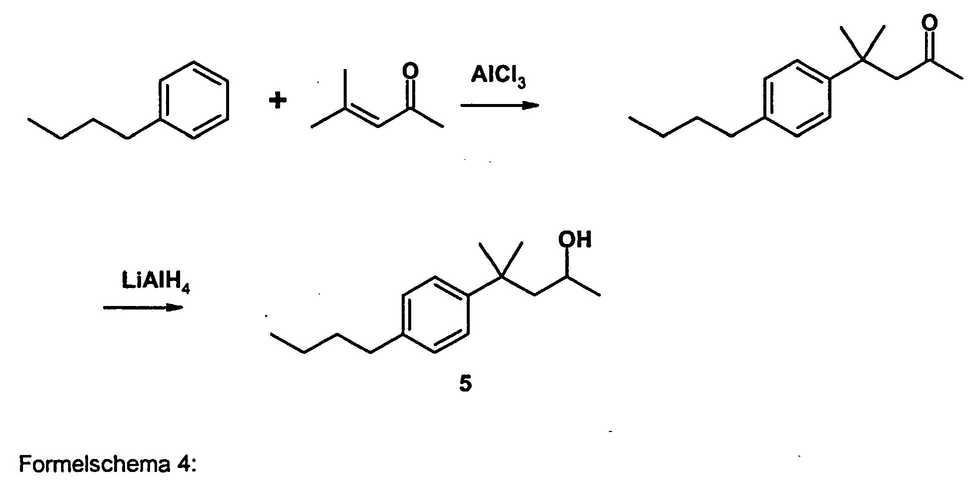

### a) Friedel-Crafts-Acylierung von n-Butylbenzol mit Mesityloxid

In einem 2l Rührwerk Kolben werden 110,4g (0,83 mol) Aluminiumchlorid vorgelegt. Anschließend werden 480 g (3,1 mol) n-Butylbenzol zugesetzt. Unter Rühren und Kühlen werden 68,6 g (0,7 mol) Mesityloxid zudosiert. Der Reaktionsansatz wird 20h nachgerührt. Anschließend wird der Reaktionsansatz analog zu Beispiel 1 weiterverarbeitet. Rohausbeute: 441g; Ausbeute an 4-Methyl-4-(4-n-butylphenyl)-2-pentanon: 60g (Reinheit: >80%)

### b) Reduktion von 4-Methyl-4-(4-n-butylphenyl)-2-pentanon mit LiAlH₄

In einem 250 ml Kolben werden 50 ml absol. THF und 0,3g LiAlH₄ (7,9 mmol) vorgelegt. Bei ca. 0°C bis 5°C tropft man 5g 4-Methyl-4-(4-n-butylphenyl)-2-pentanone (21 mmol) gelöst in 5 ml absolutem THF zu. Der Reaktionsansatz wird 1 h nachgerührt. Anschließend wird das überschüssige LiAlH₄ durch Zusatz von Wasser hydrolysiert. Nach Verseifung mit 10% iger NaOH, neutralwaschen und abdestillieren des Lösungsmittels erhält man 4g (R,S)-4-Methyl-4-p-cumenyl-2-pentanol (Reinheit: >80%)

Spektroskopische Daten von (R/S)-4-Methyl-4-(4-n-butylphenyl)-2-pentanol (Formel 5): ¹³C (CDCl₃; 75,5 MHz): δ (ppm) = 14.0 (q), 22.4 (t), 24.9 (q), 28.8 (q), 30.2 (q), 33.6 (t), 35.1 (t), 36.7 (s), 53.7 (t), 65.6 (d), 125.7 (2d), 128.4 (2d), 140.3 (s), 145.7 (s); MS: m/z (%) = 234 (4, M⁺), 176 (25), 175 (100), 161 (2), 147 (3), 131 (6), 119 (17), 105 (5), 91 (12), 77 (2), 57 (8), 45 (7), 41 (7).

### 2. Untersuchungen zur antimikrobiellen Wirkung von 4-Methyl-4-aryl-2-Pentanolen

Die Erkenntnis, dass 4-Methyl-4-aryl-2-pentanole der Formel 1 (wobei R jede der oben angegebenen Bedeutungen besitzen kann) ganz besonders zur Bekämpfung von Keimen geeignet sind, die für Körpergerüche verantwortlich sind, geht auf Untersuchungsreihen zurück, in der die besonders relevanten Keimen *Staphylococcus epidermidis, Corynebacterium xerosis* und *Brevibacterium epidermidis* untersucht wurden. Nachfolgend werden jedoch nicht nur die Ergebnisse für die Hemmung von *Staphylococcus epidermidis, Corynebacterium xerosis* und *Brevibacterium epidermidis* angegeben, sondern auch die Ergebnisse weiterer Tests. Es zeigte sich nämlich, dass die Aktivität der genannten 4-Methyl-4-aryl-2-pentanole auch gegenüber weiteren Testkeimen wie *Malassezia furfur, Trichophyton mentagrophytes, Propionibacterium acnes, Candida albicans und Aspergillus niger* überraschend hoch ist, woduch sich zusätzliche Anwendungsgebiet ergeben.

### 2.1. Allgemeine Testbedingungen:

Der Nachweis der antimikrobiellen Wirkung der gemäß den Beispielen 1-4 synthetisierten Substanzen (Formeln 2-5) erfolgte mit Hilfe des Agardilutionsverfahren in Anlehnung an DIN 58 940/ICS und DIN 58 944/ICS. Es wurden Petrischalen von 5,5 cm Durchmesser mit 8,7 ml frisch hergestelltem und bei 50 °C flüssig gehaltenem Mueller-Hinton-Agar (Merck, Art. 1.05437 bzw. Wilkins-Chalgren-Agar-Boillon, Oxoid, Art. CM 643, supplementiert mit 10 g Agar-Agar/Liter) beschickt, denen die verschiedenen Konzentrationen der verdünnten Proben in 3,3 Vol.-% = 0,3 ml zugesetzt wurden. Für den Testkeim *Malassezia furfur* wurde Mueller-Hinton-Agar verwendet, der 3% Tween80 (Merck, Art. 8.22 187) enthielt.

2,6 ml der 3,3%-igen Proben wurden mit Ethanol (96%; Merck, Art. 1.00971) verdünnt. Durch fortlaufende 2:1-Verdünnung mit Ethanol (96%-ig) wurden die weiteren Testkonzentrationen der jeweiligen Verdünnungsreihen, die in Form geometrischer Reihen angelegt wurden, hergestellt.

Durch eine weitere Verdünnung mit dem Testagar (0,3 ml Probe bzw. entsprechender Verdünnungen + 8,7 ml Agar) wurden jeweils 30-fach niedrigere Endkonzentrationen erreicht (entspricht einer Anfangskonzentration von jeweils 1100 ppm). Die im folgenden angegebenen Konzentrationen beziehen sich auf die Reinsubstanz und sind in ppm umgerechnet. Pro Testkonzentration und Nährmedium wurden 2 Agarplatten gegossen.

Es wurden folgende Untersuchungen mit jeweils 2 Agarplatten durchgeführt:

| | |
|---|---|
| K1: 9,0 ml Mueller-Hinton-Agar | (unbeimpft) |
| K2: 8,7 ml Mueller-Hinton-Agar + 0,3 ml Ethanol (96%) | (unbeimpft) |
| K3: 8,7 ml Mueller-Hinton-Agar + 0,3 ml Ethanol (96%) | (beimpft) |
| K4: 9,0 ml Mueller-Hinton-Agar | (beimpft) |
| K5: 9,0 ml Mueller-Hinton-Agar + 3% Tween80 | (unbeimpft) |
| K6: 9,0 ml Mueller-Hinton-Agar + 3% Tween80 + 0,3 ml Ethanol (96%) | (unbeimpft) |
| K7: 9,0 ml Mueller-Hinton-Agar + 3% Tween80 + 0,3 ml Ethanol (96%) | (beimpft) |
| K8: 9,0 ml Mueller-Hinton-Agar + 3% Tween80 | (beimpft) |
| K9: 9,0 ml Wilkins-Chalgren-Agar | (unbeimpft) |
| K10: 9,0 ml Wilkins-Chalgren-Agar + 0,3 ml Ethanol (96%) | (unbeimpft) |
| K11: 9,0 ml Wilkins-Chalgren-Agar + 0,3 ml Ethanol (96%) | (beimpft) |
| K12: 9,0 ml Wilkins-Chalgren-Agar | (beimpft) |

Nach Verfestigung und Trocknung (ca. 1 h bei 37 °C) wurden die Testplatten punktförmig mit jeweils 1 µl der in den nachfolgenden Beispielen aufgeführten Testkeimsuspensionen beimpft. Zur Überprüfung von Reinheit und Identität wurden die aerob wachsenden Bakterien (*Brevibacterium epidermidis, Corynebakterium xerosis, Staphylococcus aureus; Staphylococcus epidermidis*) auf Columbia Blut-Agar (BioMérieux, Art. 43049). Der Schimmelpiz Aspergillus niger, die Hefe Candida albicanns und der Hautpilz *Trichophyton mentagrophytes* wurden auf Sabouraud-Agar (BioMerieux, Art. 43555) kultiviert. *Malassezia furfur* wurde auf Sabourad-HLT-Agar mit Enthemmern (Zusatz von Tween80: 1%; Lecithin: 0,3%; Histidin: 0,1%; Merck, Art. 1.18368) angezüchtet. *Propionibacterium acnes* wurde auf Schaedler-Agar (BioMérieux, Art. 43273) kultiviert. Weitere Angaben zu den Testkeimen sind Tabelle 1 zu entnehmen.

**Tabelle 1: Testkeime (Stammbezeichnungen) und Keimzahlen**

| Testkeim | Stammbez. | KBE*/ml |
|---|---|---|
| | | |
| *Brevibacterium epidermidis* | ATCC 35514 | 2,8 x 10⁷ |
| *Corynebacterium xerosis* | ATCC 7711 | 1,9 x 10⁷ |
| *Propionibacterium acnes* | ATCC 11829 | 2,3 x 10⁸ |
| *Staphylococcus epidermidis* | ATCC12228 | 2,9 x 10⁷ |
| *Malassezia furfur* | DSM 6171 | 2,8 x 10⁷ |
| *Aspergillus niger* | ATCC 16404 | 1,7 x 10⁷ |
| *Trichophyton mentagrophtes* | CBS 26379 | 2,5 x 10⁷ |
| *Candida albicanns* | ATCC 10231 | 2,0 x 10⁷ |
| | | |
| KBE* = koloniebildende Einheiten | | |

Die Herstellung der Testkeimsuspensionen der aerob wachsenden bakteriellen Keime erfolgte durch Bebrütung von Mueller-Hinton-Bouillon (Merck, Art. 1.10293) bei 36 °C, die mit wenigen Einzelkolonien der jeweiligen Testkeime beimpft worden war. Nach dem Erreichen einer deutlichen Trübung wurde den Suspensionen so viel sterile Nährbouillon zugegeben, dass deren Trübung dem McFarland Standard 0,5 entsprach (ca. 1,5 x 10⁸ KBE/ml).

Zur Herstellung der übrigen Testkeimsuspensionen wurden die Teststämme auf den oben genannten, festen Nährmedium kultiviert, mittels sterilem Tupfer abgeerntet und in so viel Mueller-Hinton-Bouillon aufgenommen bzw. verdünnt, dass die Trübung der Suspensionen dem McFarland Standard 0,5 entsprach.

Alle Testkeimsuspensionen, mit Ausnahme von *Propionibacterium acnes*, wurden nochmals mit steriler Bouillon 1:10 verdünnt und deren Keimzahl im Oberflächenverfahren per Spiralometer ermittelt (Ergebnisse: siehe Tabelle 1).

Die inokulierten Platten wurden unter den in Tabelle 2 angegebenen Bedingungen bebrütet und anschließend ausgewertet. Als MHK (Minimale Hemmkonzentration) wurde die niedrigste Wirkstoffkonzentration angesehen, bei der makroskopisch kein Wachstum vorhanden ist. Minimales, kaum sichtbares Wachstum oder wenige kleine Einzelkolonien wurden als Hemmung bewertet.

**Tabelle 2: Inokulation und Bebrütung**

| Testkeim | Stamm-Bez. | Wachstumsbedingungen | Nährmedium | Bebrütung |
|---|---|---|---|---|
| | | | | |
| *Brevibacterium epidermidis* | ATCC 35514 | Aerob | Mueller-Hinton-Agar | 18h bei 36 °C |
| *Corynebacterium xerosis* | ATCC 7711 | Aerob | Mueller-Hinton-Agar | 18h bei 36 °C |
| *Propionibacterium acnes* | ATCC 11829 | Anaerob | Wilkins-Chalgren-Agar | 72h bei 36 °C |
| *Candida albicans* | ATCC 10231 | Aerob | Mueller-Hinton-Agar | 48h bei 30 °C |
| *Staphylococcus epidermidis* | ATCC 12228 | Aerob | Mueller-Hinton-Agar | 18h bei 36°C |
| *Malassezia furfur* | DSM 6171 | Aerob | Mueller-Hinton-Agar + 3% Tween 80 | 72h bei 30 °C |
| *Trichophyton mentagrophytes* | CBS 26379 | Aerob | Mueller-Hinton-Agar | 72h bei 30 °C |
| *Aspergillus niger* | ATCC 16404 | Aerob | Mueller-Hinton-Agar | 48h bei 30 °C |

### 2. 2. MHK-Werte von (R/S)-4-Methyl-4-arvl-2-pentanolen

Die MHK-Werte verschiedener (R/S)4-Methyl-4-aryl-2-pentanole wurden gemäß den unter 2.1. beschriebenen allgemeinen Testbedingungen bestimmt (vgl. Tabelle 3; MHK-Werte für (R/S-4-Methyl-4-phenyl-2-pentanol, für (R/S)-4-Methyl-4-p-tolyl-2-pentanol, (R/S)-4-Methyl-4-p-cumenyl-2-pentanol und (R/S)-4-Methyl-4-(4-n-butylphenyl)-2-pentanol).

| Tabelle 3: | | | | |
|---|---|---|---|---|
| **MHK-Werte für (R/S)-4-Methyl-4-phenyl-2-pentanol 2; (R/S)-4-Methyl-4-tolyl-2-pentanol 3;** | | | | |
| **(R/S)-4-Methyl-4-p-cumenyl-2-pentanol 4 und (R/S)-4-Methyl-4-(4-n-butylphenyl)-2-pentanol 5** | | | | |
| | | | | |
| Mikroorganismus | 2 | 3 | 4 | 5 |
| | | | | |
| *Staphylococcus epidermidis* | 1100 | 550 | 138 | 1100 |
| *Corynebacterium xerosis* | 1100 | 275 | 69 | 69 |
| *Brevibacterium epidermidis* | 1100 | 550 | 138 | 550 |
| *Propionibacterium acnes* | 1100 | 550 | 138 | >1100,1 |
| *Malassezia furfur* | 69 | 69 | 550 | 550 |
| *Trichophyton mentagrophytes* | 550 | 138 | 69 | 69 |
| *Candida albicans* | 1100 | 1100 | >1100 | >1100 |
| *Aspergillus niger* | 550 | 550 | >1100 | >1100 |
| *Escherichia coli* | >1100 | >1100 | >1100 | >1100 |
| *Pseudomonas aeruginosa* | >1100 | >1100 | >1100 | >1100 |

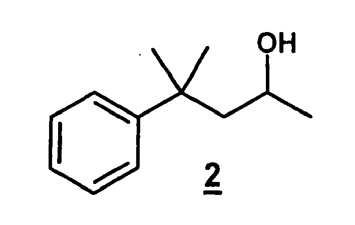

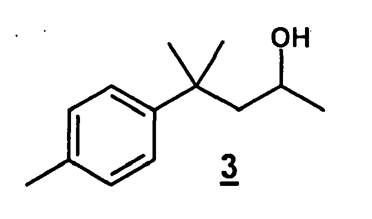

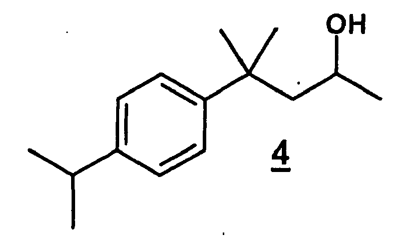

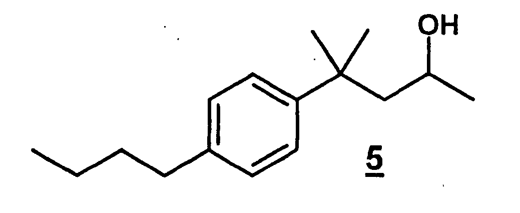

Eine deutliche Hemmung des Wachstums grampositiver, Körpergeruch verursachender Bakterien wie *Staphylococcus epidermidis, Corynebacterium xerosis, Brevibacterium epidermidis* bereits in Konzentrationen kleiner 0,15% konnte für alle in Tabelle 3 gelisteten Substanzen beobachtet werden. Die stärkste Wirksamkeit zeigte hierbei das (R/S)-4-Methyl-4-p-cumenyl-2-propanol (*Staphylococcus epidermidis:* 138ppm = 138µg/ml; *Corynebacterium xerosis:* 69ppm = 69µg/ml; *Brevibacterium epidermidis:* 138ppm = 138 µg/ml). Die Wirksamkeit gegenüber dem für Akne verantworlichen Pro*pionibacterium acnes* ist ebenfalls bei (R/S)-4-Methyl-4-p-cumenyl-2-propanol besonders ausgeprägt (138ppm = 138µg/ml), obgleich auch bei den Substanzen 2, 3 und 5 eine Wirksamkeit zu beobachten ist. Gegenüber Hefen wie Candida albicans und Schimmelpilzen sind hingegen das (R/S)-4-Methyl-4-phenyl-2-propanol (Formel 2) und das (R/S)-4-Methyl-4-p-tolyl-2-propanol die wirksamsten Substanzen. Da die genannten Substanzen oder auch Gemische der Substanzen über ein sehr breites Wirksamkeitsspekturm verfügen, können Sie auch als Konservierungsmitteln Einsatz finden.

Es sei darauf hingewiesen, dass der allg. Begriff (R/S)-4-Methyl-4-aryl-2-pentanol (Formel 1) im Rahmen des vorliegenden Textes sowohl die 2S-konfigurierten Enantiomere als auch die 2R-konfigurierten Enantiomere sowie beliebige Mischungen aus 2S- und 2R-konfigurierten 4-Methyl-4-aryl-2-pentanolen umfasst. Aus kommerziellen Gründen ist es zwar besonders vorteilhaft, die Razemate der jeweiligen Alkohole zur Hemmung des Wachstums von Mikroorganismen einzusetzen, da diese synthetisch besonders leicht zugänglich sind. Die reinen Enantiomere oder nicht-razemische Mischungen dieser Enantiomere sind aber ebenfalls für die erfindungsgemäßen Zwecke geeignet.

Ein wichtiger Anwendungsbereich von (R/S)-4-Methyl-4-aryl-2-pentanolen der Formel 1 ist die Hemmung der für die Bildung von Körpergeruch (inkl. Achsel- und Fußgeruch) verantwortlichen Bakterien (insbesondere *Staphylococcus-, Corynebacterium-* und *Brevibacterium-Arten*). Darüberhinaus können (R/S)-4-Methyl-4-aryl-2-pentanole zur Hemmung Mykosen verursachender Haut- und Nagelpilze (Dermatomykosen, Nagelmykosen; *Trichophyton-* und *Epidermophyton*-Arten), zur Hemmung für Schuppenbildung verantwortlicher Mikroorganismen (*Malassezia furfur*, Syn.; *Pityrosporum ovale* oder *P. orbiculare*) und zur Behandlung von Akne (Hemmung des Wachstums von *Propioibacterium acnes*) eingesetzt werden. Eine Wirksamkeit auch gegenüber Pilzen wie *Aspergillus niger* und Hefen wie *Candida albicans*, sowie in Konzentrationen >1100 ppm auch gegenüber gramnegativen Bakterien wie z.B. *Escherichia coli* oder *Pseudomonas aeruginosa* erlaubt darüber hinaus auch eine Verwendung der beschriebenen (R/S)-4-Methyl-4-aryl-2-pentanole der. Formel 1 als Konservierungsmittel.

Ein (a) antimikrobielles (R/S)-4-Methyl-4-aryl-2-pentanol der Formel 1 oder (b) mehrere Substanzen der Formel 1 enthaltende Wirkstoffmischungen werden, insbesondere soweit sie gegen Körpergeruch verursachende Keime eingesetzt werden, in der Regel topisch in Form von Lösungen, Cremes, Lotionen, Gelen, Sprays o.dgl. appliziert. Für andere Zwecke ist in manchen Fällen eine orale (Tabletten, Kapseln, Pulver, Tropfen), intravenöse, intraoculare, intraperitoneale oder intramuskuläre Applikation oder eine Applikation in Form eines imprägnierten Verbands sinnvoll.

Die Konzentration der Wirkstoffe (R/S)-4-Methyl-4-aryl-2-pentanole der Formel 1 in den (topisch) applizierenden Formulierungen liegt vorzugsweise im Bereich von 0,0069% - 20 Gew.-% und bevorzugt im Bereich von 0,05%-0,5 Gew.-%. Der antimikrobielle Wirkstoffkomplex kann hierbei (a) prophylaktisch oder (b) im Bedarfsfall zum Einsatz kommen.

Die Konzentration der z.B. täglich zu applizierenden Wirkstoffmenge ist unterschiedlich und hängt vom physiologischen Zustand des Probanden sowie individualspezifischen Parametern wie Alter oder Körpergewicht ab. (R/S)-4-Methyl-4-aryl-2-pentanole der Formel 1 können sowohl allein, als Gemische oder auch in Kombination mit weiteren antimikrobiell wirksamen Substanzen zum Einsatz gelangen.

## Patentansprüche

1. Verbindung der Formel 1 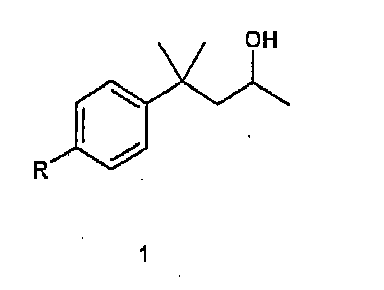 mitR=
- Hydroxy,
- Alkoxygruppe mit bis zu 10 C-Atomen,
- geradkettiges oder verzweigtes, gesättigtes oder ungesättigtes Alkyl mit bis zu 10 C-Atomen, jedoch nicht i-Propyl,
- Alkylthioethergruppe mit bis zu 10 C-Atomen, wobei die Alkylthioethergruppe über eine Thioetherbrücke mit dem aromatischen Ring verbunden ist,
- Fluor, Chlor, Brom, lod oder
- durch ein oder mehrere Sauerstoff- und/oder Schwefelatome unterbrochenes Alkyl mit bis zu 10 C-Atomen

2. Verwendung einer Verbindung der Formel 1 mit R =
- Wasserstoff,
- Hydroxy,
- Alkoxygruppe mit bis zu 10 C-Atomen,
- geradkettiges oder verzweigtes, gesättigtes oder ungesättigtes Alkyl mit bis zu 10 C-Atomen,
- Alkylthioethergruppe mit bis zu 10 C-Atomen, wobei die Alkylthioethergruppe über eine Thioetherbrücke mit dem aromatischen Ring verbunden ist,
- Fluor, Chlor, Brom, lod oder
- durch ein oder mehrere Sauerstoff- und/oder Schwefelatome unterbrochenes Alkyl mit bis zu 10 C-Atomen
als antimikrobiefler Wirkstoff
(a) zur kosmetischen Behandlung von Schuppen verursachenden Mikroorganismen,
(b) zur kosmetischen Behandlung von Körpergeruch verursachenden Mikroorganismen,
(c) zur kosmetischen Behandlung von Akne verursachenden Mikroorganismen
(d) zur kosmetischen Behandlung von Mykosen verursachenden Mikroorganismen.
(e) zur Behandlung von Mikroorganismen auf oder in unbelebter Materie,
und/oder
(f) zur Konservierung verderblicher Artikel.

3. Verwendung einer Verbindung der Formel 1
mit R =
- Wasserstoff,
- Hydroxy,
- Alkoxygruppe mit bis zu 10 C-Atomen,
- geradkettiges oder verzweigtes, gesättigtes oder ungesättigtes Alkyl mit bis zu 10 C-Atomen,
- Alkylthioethergruppe mit bis zu 10 C-Atomen, wobei die Alkylthloethergruppe über eine Thioetherbrücke mit dem aromatischen Ring verbunden ist,
- Fluor, Chlor, Brom, lod oder
- durch ein oder mehrere Sauerstoff- und/oder Schwefelatome unterbrochenes Alkyl mit bis zu 10 C-Atomen
zur Herstellung einer antimikrobiellen kosmetischen oder einer antimikrobiellen pharmazeutischen Formulierung.

4. Verfahren zur kosmetischen Behandlung von (a) Schuppen verursachenden Mikroorganismen, (b) Körpergeruch verursachenden Mikroorganismen (c) Akne verursachenden Mikroorganismen und/oder (d) Mykosen verursachenden Mikroorganismen, umfassend die topische Applikation einer antimikrobiell wirksamen Menge einer oder mehrerer Verbindungen der Formel 1 mit R =
- Wasserstoff,
- Hydroxy,
- Alkoxygruppe mit bis zu 10 C-Atomen,
- geradkettiges oder verzweigtes, gesättigtes oder ungesättigtes Alkyl mit bis zu 10 C-Atomen,
- Alkylthioethergruppe mit bis zu 10 C-Atomen, wobei die Alkylthioethergruppe über eine Thioetherbrücke mit dem aromatischen Ring verbunden ist,
- Fluor, Chlor, Brom, lod oder
- durch ein oder mehrere Sauerstoff- und/oder Schwefelatome unterbrochenes Alkyl mit bis zu 10 C-Atomen
auf den menschlichen oder tierischen Körper.

5. Antimikrobielle Zusammensetzung, umfassend die folgenden Komponenten:
(a) eine antimikrobiell wirksame Menge einer oder mehrerer Verbindungen der Formel 1 mit R =
- Hydroxy,
- Alkoxygruppe mit bis zu 10 C-Atomen,
- geradkettiges oder verzweigtes, gesättigtes oder ungesättigtes Alkyl mit bis zu 10 C-Atomen,
- Alkylthioethergruppe mit bis zu 10 C-Atomen, wobei die Alkylthioethergruppe über' eine Thioetherbrücke mit dem aromatischen Ring verbunden ist,
- Fluor, Chlor, Brom, lod oder
- durch ein oder mehrere Sauerstoff- und/oder Schwefelatome unterbrochenes Alkyl mit bis zu 10 C-Atomen
sowie
(b) eine zu Komponente (a) kompatible Trägersubstanz.

6. Duftstoffkomposition, umfassend
(a) eine sensorisch wirksame Menge eines Duftstoffes,
(b) eine konservierend wirkende Menge einer oder mehrerer Verbindungen der Formel 1 mit R =
- Hydroxy,
- Alkoxygruppe mit bis zu 10 C-Atomen,
- geradkettiges oder verzweigtes, gesättigtes oder ungesättigtes Alkyl mit bis zu 10 C-Atomen,
- Alkylthioethergruppe mit bis zu 10 C-Atomen, wobei die Alkylthioethergruppe über eine Thioetherbrücke mit dem aromatischen Ring verbunden ist,
- Fluor, Chlor, Brom, lod oder
- durch ein oder mehrere Sauerstoff- und/oder Schwefelatome unterbrochenes Alkyl mit bis zu 10 C-Atomen
und
(c) gegebenenfalls einen oder mehrere Trägerstoffe und/oder Zusatzstoffe.

7. Kosmetikprodukt gegen Achsel- und Fußgeruch verursachende Keime, umfassend eine antimikrobiell wirksame Menge einer oder mehrerer Verbindungen der Formel 1 mit R =
- Wasserstoff,
- Hydroxy,
- Alkoxygruppe mit bis zu 10 C-Atomen,
- geradkettiges oder verzweigtes, gesättigtes oder ungesättigtes Alkyl mit bis zu 10 C-Atomen,
- Alkylthioethergruppe mit bis zu 10 C-Atomen, wobei die Alkylthiaethergruppe über eine Thioetherbrücke mit dem aromatischen Ring verbunden ist,
- Fluor, Chlor, Brom, lod oder
- durch ein oder mehrere Sauerstoff- und/oder Schwefelatome unterbrochenes Alkyl mit bis zu 10 C-Atomen,
wobei eine Mischung von 1 % 4-Methyl-4-phenyl-2-pentanol in 50%igem Propylenglykol ausgenommen ist.

## Claims

1. Compound of the formula 1 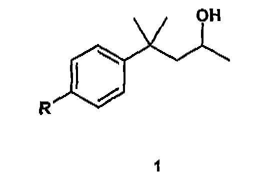 where R =
- hydroxyl,
- alkoxy group with up to 10 C atoms,
- straight-chain or branched, saturated or unsaturated alkyl with up to 10 C atoms, but not i-propyl,
- alkylthioether group with up to 10 C atoms, the alkylthioether group being bonded to the aromatic ring via a thioether bridge,
- fluorine, chlorine, bromine, iodine, or
- alkyl with up to 10 C atoms that is interrupted by one or more oxygen and/or sulfur atoms.

2. Use of a compound of the formula 1 where R =
- hydrogen,
- hydroxyl,
- alkoxy group with up to 10 C atoms,
- straight-chain or branched, saturated or unsaturated alkyl with up to 10 C atoms,
- alkylthioether group with up to 10 C atoms, the alkylthioether group being bonded to the aromatic ring via a thioether bridge,
- fluorine, chlorine, bromine, iodine, or
- alkyl with up to 10 C atoms that is interrupted by one or more oxygen and/or sulfur atoms,
as an antimicrobial active substance
(a) for the cosmetic treatment of microorganisms causing dandruff,
(b) for the cosmetic treatment of microorganisms causing body odour,
(c) for the cosmetic treatment of microorganisms causing acne,
(d) for the cosmetic treatment of microorganisms causing mycoses,
(e) for the treatment of microorganisms on or in inanimate material,
and/or
(f) for the preservation of perishable articles.

3. Use of a compound of the formula 1
where R =
- hydrogen,
- hydroxyl,
- alkoxy group with up to 10 C atoms,
- straight-chain or branched, saturated or unsaturated alkyl with up to 10 C atoms,
- alkylthioether group with up to 10 C atoms, the alkylthioether group being bonded to the aromatic ring via a thioether bridge,
- fluorine, chlorine, bromine, iodine, or
- alkyl with up to 10 C atoms that is interrupted by one or more oxygen and/or sulfur atoms,
for the preparation of an antimicrobial cosmetic or an antimicrobial pharmaceutical formulation.

4. Method for the cosmetic treatment of (a) microorganisms causing dandruff, (b) microorganisms causing body odour, (c) microorganisms causing acne and/or (d) microorganisms causing mycoses, comprising the topical application of an antimicrobially active amount of one or more compounds of the formula 1 where R =
- hydrogen,
- hydroxyl,
- alkoxy group with up to 10 C atoms,
- straight-chain or branched, saturated or unsaturated alkyl with up to 10 C atoms,
- alkylthioether group with up to 10 C atoms, the alkylthioether group being bonded to the aromatic ring via a thioether bridge,
- fluorine, chlorine, bromine, iodine, or
- alkyl with up to 10 C atoms that is interrupted by one or more oxygen and/or sulfur atoms
onto the human or animal body.

5. Antimicrobial composition, comprising the following components:
(a) an antimicrobially active amount of one or more compounds of the formula 1 where R =
- hydroxyl,
- alkoxy group with up to 10 C atoms,
- straight-chain or branched, saturated or unsaturated alkyl with up to 10 C atoms,
- alkylthioether group with up to 10 C atoms, the alkylthioether group being bonded to the aromatic ring via a thioether bridge,
- fluorine, chlorine, bromine, iodine, or
- alkyl with up to 10 C atoms that is interrupted by one or more oxygen and/or sulfur atoms
as well as
(b) a carrier substance compatible with component (a).

6. Perfume composition, comprising
(a) a perfume in an amount that has a sensory effect,
(b) one or more compounds of the formula 1 where R =
- hydroxyl,
- alkoxy group with up to 10 C atoms,
- straight-chain or branched, saturated or unsaturated alkyl with up to 10 C atoms,
- alkylthioether group with up to 10 C atoms, the alkylthioether group being bonded to the aromatic ring via a thioether bridge,
- fluorine, chlorine, bromine, iodine, or
- alkyl with up to 10 C atoms that is interrupted by one or more oxygen and/or sulfur atoms
in an amount that has a preservative action
and
(c) optionally one or more excipients and/or additives.

7. Cosmetic product against microbes that cause underarm and foot odour comprising an antimicrobially active amount of one or more compounds of the formula 1 where R =
- hydrogen,
- hydroxyl,
- alkoxy group with up to 10 C atoms,
- straight-chain or branched, saturated or unsaturated alkyl with up to 10 C atoms,
- alkylthioether group with up to 10 C atoms, the alkylthioether group being bonded to the aromatic ring via a thioether bridge,
- fluorine, chlorine, bromine, iodine, or
- alkyl with up to 10 C atoms that is interrupted by one or more oxygen and/or sulfur atoms,
a mixture of 1% 4-methyl-4-phenyl-2-pentanol in 50% strength propylene glycol being excepted.

## Revendications

1. Composé de la formule 1 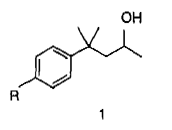 avec R =
- un groupe hydroxy,
- un groupe alcoxy avec jusqu'à 10 atomes de C,
- un groupe alkyle linéaire ou ramifié, saturé ou insaturé avec jusqu'à 10 atomes de C, toutefois pas le groupe i-propyle,
- un groupe alkylthioéther avec jusqu'à 10 atomes de C, le groupe alkylthioéther étant lié par l'intermédiaire d'un pont thioéther au noyau aromatique,
- un atome de fluor, de chlore, de brome, d'iode ou
- un groupe alkyle interrompu par un ou plusieurs atomes d'oxygène et/ou d'azote avec jusqu'à 10 atomes de C.

2. Utilisation d'un composé de la formule 1 avec R =
- un atome d'hydrogène,
- un groupe hydroxy,
- un groupe alcoxy avec jusqu'à 10 atomes de C,
- un groupe alkyle linéaire ou ramifié, saturé ou insaturé avec jusqu'à 10 atomes de C,
- un groupe alkylthioéther avec jusqu'à 10 atomes de C, le groupe alkylthioéther étant lié par l'intermédiaire d'un pont thioéther au noyau aromatique,
- un atome de fluor, de chlore, de brome, d'iode ou
- un groupe alkyle interrompu par un ou plusieurs atomes d'oxygène et/ou de soufre avec jusqu'à 10 atomes de C,
comme matière active antimicrobienne
(a) pour le traitement cosmétique de microorganismes occasionnant des pellicules,
(b) pour le traitement cosmétique de microorganismes occasionnant des odeurs corporelles,
(c) pour le traitement cosmétique de microorganismes occasionnant l'acné,
(d) pour le traitement cosmétique de microorganismes occasionnant des mycoses,
(e) pour le traitement de microorganismes sur ou dans un matériau sans vie,
et/ou
(f) pour la conservation d'articles perissables.

3. Utilisation d'un composé de la formule (1) avec R =
- un atome d'hydrogène,
- un groupe hydroxy,
- un groupe alcoxy avec jusqu'à 10 atomes de C,
- un groupe alkyle linéaire ou ramifié, saturé ou insaturé avec jusqu'à 10 atomes de C,
- un groupe alkylthioéther avec jusqu'à 10 atomes de C, le groupe alkylthioéther étant lié par l'intermédiaire d'un pont thioéther au noyau aromatique,
- un atome de fluor, de chlore, de brome, d'iode ou
- un groupe alkyle interrompu par un ou plusieurs atomes d'oxygène et/ou de soufre avec jusqu'à 10 atomes de C,
pour la préparation d'une formulation cosmétique antimicrobienne ou pharmaceutique antimicrobienne.

4. Procédé pour le traitement cosmétique (a) de microorganismes occasionnant des pellicules, (b) de microorganismes occasionnant une odeur corporelle, (c) de microorganismes occasionnant l'acné et/ou (d) de microorganismes occasionnant des mycoses comprenant l'application topique d'une quantité active d'un point de vue antimicrobien d'un ou plusieurs composés de la formule (1) avec R =
- un atome d'hydrogène,
- un groupe hydroxy,
- un groupe alcoxy avec jusqu'à 10 atomes de C,
- un groupe alkyle linéaire ou ramifié, saturé ou insaturé avec jusqu'à 10 atomes de C,
- un groupe alkylthioéther avec jusqu'à 10 atomes de C, le groupe alkylthioéther étant lié par l'intermédiaire d'un pont thioéther au noyau aromatique,
- un atome de fluor, de chlore, de brome, d'iode ou
- un groupe alkyle interrompu par un ou plusieurs atomes d'oxygène et/ou de soufre avec jusqu'à 10 atomes de C,
sur le corps d'un être humain ou d'un animal.

5. Composition antimicrobienne comprenant les constituants suivants :
(a) une quantité efficace d'un point de vue antimicrobien d'un ou plusieurs composés de la formule 1 avec R =
- un groupe hydroxy,
- un groupe alcoxy avec jusqu'à 10 atomes de C,
- un groupe alkyle linéaire ou ramifié, saturé ou insaturé avec jusqu'à 10 atomes de C,
- un groupe alkylthioéther avec jusqu'à 10 atomes de C, le groupe alkylthioéther étant lié par l'intermédiaire d'un pont thioéther au noyau aromatique,
- un atome de fluor, de chlore, de brome, d'iode ou
- un groupe alkyle interrompu par un ou plusieurs atomes d'oxygène et/ou de soufre avec jusqu'à 10 atomes de C,
ainsi que
(b) une suspension de support compatible au constituant (a).

6. Composition de matière odorante comprenant
(a) une quantité sensoriellement active d'une matière odorante,
(b) une quantité active d'un point de vue de la conservation d'un ou plusieurs composés de la formule 1 avec R =
- un groupe hydroxy,
- un groupe alcoxy avec jusqu'à 10 atomes de C,
- un groupe alkyle linéaire ou ramifié, saturé ou insaturé avec jusqu'à 10 atomes de C,
- un groupe alkylthioéther avec jusqu'à 10 atomes de C, le groupe alkylthioéther étant lié par l'intermédiaire d'un pont thioéther au noyau aromatique,
- un atome de fluor, de chlore, de brome, d'iode ou
- un groupe alkyle interrompu par un ou plusieurs atomes d'oxygène et/ou de soufre avec jusqu'à 10 atomes de C,
et
(c) éventuellement une ou plusieurs matières de support et/ou additifs.

7. Produit cosmétique contre des microbes occasionnant une odeur sous les aisselles et des pieds comprenant
une quantité active d'un point de vue antimicrobien d'un ou plusieurs composés de la formule 1 avec R =
- un atome d'hydrogène,
- un groupe hydroxy,
- un groupe alcoxy avec jusqu'à 10 atomes de C,
- un groupe alkyle linéaire ou ramifié, saturé ou insaturé avec jusqu'à 10 atomes de C,
- un groupe alkylthioéther avec jusqu'à 10 atomes de C, le groupe alkylthioéther étant lié par l'intermédiaire d'un pont thioéther au noyau aromatique,
- un atome de fluor, de chlore, de brome, d'iode ou
- un groupe alkyle interrompu par un ou plusieurs atomes d'oxygène et/ou de soufre avec jusqu'à 10 atomes de C,
un mélange de 1 % de 4-méthyl-4-phényl-2-pentanol dans du propylèneglycol à 50 % étant exclu.
